# EUROPEAN PATENT APPLICATION

(11) **EP 4 257 119 A1**
(43) Date of publication of application: **11.10.2023**
(21) Application number: 21900988.3
(22) Date of filing: 01.12.2021
(51) Int. Cl.: A61K 9/16, A61K 47/12, A61K 31/502, A61P 35/00

(54) **OLAPARIB SOLID DISPERSION COMPOSITION WITH IMPROVED STABILITY AND BIOAVAILABILITY**

(30) Priority: 01.12.2020 KR 20200166029
(71) Applicant: Samyang Holdings Corporation, Seoul 03129 (KR)
(72) Inventor: LIM, Hye Jung, Seongnam-si Gyeonggi-do 13490 (KR); PARK, Sang Yeob, Yongin-si Gyeonggi-do 16875 (KR); WON, Woong Roeck, Seoul 04424 (KR)
(74) Representative: Dietz, Christopher Friedrich
(86) International application number: PCT/KR2021/017964
(87) International publication number: WO 2022/119300

(57) **Abstract**

The present invention relates to an olaparib solid dispersion composition having improved stability and bioavailability, and more specifically, to: an olaparib solid dispersion; and an oral composition comprising same, wherein the olaparib solid dispersion includes olaparib, which is a poorly soluble drug, a polymethacrylate copolymer, and a cellulose-based polymer as essential components, has excellent storage stability and thermal stability, and exhibits uniform drug releasability under all of the various pH conditions inside the body.

## Description

### TECHNICAL FIELD

The present invention relates to a solid dispersion composition of Olaparib with enhanced solubility and bioavailability, and more specifically, it relates to an Olaparib solid dispersion and an oral composition comprising the same, wherein the solid dispersion comprises Olaparib that is a poorly water-soluble drug, polymethacrylate copolymer and cellulose-based polymer as essential components, and the solid dispersion has excellent storage stability and thermal stability, exhibits uniform drug releasability under all of the various pH conditions in the body, and has enhanced bioavailability.

### BACKGROUND ART

Olaparib is a phthalazinone derivative drug and the first PARP inhibitor approved in 2014, and it has been used for treatment of breast cancer and ovarian cancer. Olaparib is weak acidic with pKa 12.07 and is a poorly water-soluble substance with very low water solubility (0.1 to 0.13 mg/ml) in the physiological pH range, and its permeability is not so high and it is known as falling in BCS class 4. Such low solubility and bioavailability of the active drug ingredient make the drug formulation problematic, and so various methods for improvement such as crystal form change or salt form preparation, etc. have been tried.

Lynparza^{™} capsule which is a commercial formulation product, uses Gelucire^{™} 44/14 which is a lipid excipient, to improve the drug solubility. However, it has the drug content of 10 % in the formulation wherein 50 mg of Olaparib is filled in hard capsule of 0 size, and thus in case of 400 mg therapeutic dose, 8 capsules should be taken at once inconveniently.

In order to improve the solubility of poorly water-soluble drug, various methods have been attempted, such as modification of the drug itself such as salt or crystal form, prodrug preparation, etc., micronization or nanoparticulation through particle size reduction of the drug, micelle formation, etc. However, in improving such properties, because drug characteristics such as solubility, pH, pKa, melting point, molecular weight, functional group, three-dimensional structure, crystallinity, permeability, absorption site, decomposition rate, effective drug capacity, etc., are different drug by drug, one technology cannot be universally applied to all drugs, and it is necessary to find a method that is suitable for a specific drug.

From this point of view, in order to improve medication compliance of patients for Olaparib, various researches are now in progress to reduce the dosage unit by increasing the bioavailability and increasing the drug content in the formulation at the same time.

### CONTENTS OF THE INVENTION

### PROBLEMS TO BE SOLVED

The purpose of the present invention is to provide a solid dispersion comprising Olaparib, which has excellent storage stability and thermal stability, exhibits uniform drug releasability under all of the various pH conditions in the body, and has enhanced bioavailability, and a method for preparing the same.

The other purpose of the present invention is to provide an oral composition comprising Olaparib, which has excellent storage stability and thermal stability, exhibits uniform drug releasability under all of the various pH conditions in the body, and has enhanced bioavailability, and a method for preparing the same.

### TECHNICAL MEANS

One aspect of the present invention relates to a solid dispersion comprising Olaparib; polymethacrylate copolymer; and cellulose-based polymer.

In an embodiment, the polymethacrylate copolymer is a cationic polymer with dimethyl-aminoethyl methacrylate.

In an embodiment, the polymethacrylate copolymer is poly(butyl methacrylate-co-(2-dimethylaminoethyl) methacrylate-co-methyl methacrylate).

In an embodiment, the cellulose-based polymer is selected from the group consisting of hydroxypropylmethylcellulose, microcrystalline cellulose, hydroxypropylcellulose, carboxymethylcellulose, ethylcellulose, cellulose acetate, methylcellulose and mixtures thereof.

In an embodiment, the solid dispersion further comprises D-α-tocopheryl polyethylene glycol succinate.

In an embodiment, the solid dispersion is obtained by spray drying.

In an embodiment, the solid dispersion comprises amorphous Olaparib.

The other aspect of the present invention relates to a method for preparing a solid dispersion, the method comprising: (1) a step of dissolving or dispersing Olaparib together with polymethacrylate copolymer in a solvent; (2) a step of adding cellulose-based polymer to the solution or dispersion liquid obtained in said step (1) and mixing them; and (3) a step of removing the solvent from the solution or dispersion liquid obtained in said step (2).

In an embodiment, the removal of the solvent is conducted by spray drying.

Another aspect of the present invention relates to an oral composition comprising the solid dispersion; pharmaceutically acceptable organic acid; and pharmaceutically acceptable diluent.

In an embodiment, the pharmaceutically acceptable organic acid is selected from the group consisting of acrylic acid, tartaric acid, citric acid, malic acid, maleic acid, fumaric acid, acetic acid, succinic acid, lactic acid, succinic acid, malonic acid, glutaric acid and mixtures thereof.

In an embodiment, the pharmaceutically acceptable diluent is water-soluble diluent.

In an embodiment, the water-soluble diluent is selected from the group consisting of sugar alcohols, saccharides and mixtures thereof.

In an embodiment, the oral composition is for treatment of cancer.

Still another aspect of the present invention relates to a method for preparing an oral composition comprising a step of mixing the solid dispersion with pharmaceutically acceptable organic acid and pharmaceutically acceptable diluent.

### EFFECT OF THE INVENTION

The solid dispersion and the oral composition comprising the same disclosed in the present invention are capable of making Olaparib amorphized stably, and thus having excellent storage stability and thermal stability and exhibiting uniform drug releasability under all of the various pH conditions in the body, and increasing the solubility and dissolution rate of Olaparib, and thereby improving *in vivo* absorption ratio of the drug and enhancing bioavailability. In addition, as compared with commercial product, the drug content in the formulation is increased by reducing use of additive, and thereby the number of dosing unit taken at once is reduced so that the medication compliance of patients can be improved, and additional advantages are expected in terms of the productivity such as preparation and packaging, etc. through application of composition and production process more favorable for formulation.

### BRIEF EXPLANATION OF THE DRAWINGS

Figure 1 represents the XRPD patterns of the solid dispersions prepared in Examples 1 and 2 and Comparative Example 1, respectively, and Olaparib API (active pharmaceutical ingredient), showing that Olaparib API itself used for preparing the solid dispersions was crystalline whereas the Olaparib contained in all solid dispersions prepared in Examples 1 and 2 and Comparative Example 1 was amorphous.
Figure 2 represents the elution patterns of the tablet prepared in Example 3 in pH 1.2 solution, pH 4.0 solution, pH 6.8 solution and water (distilled water, DW).
Figure 3 represents the elution patterns of the tablet prepared in Example 4 in pH 1.2 solution, pH 4.0 solution, pH 6.8 solution and water (distilled water, DW).
Figure 4 represents the elution patterns of the tablet prepared in Comparative Example 2 in pH 1.2 solution, pH 4.0 solution, pH 6.8 solution and water (distilled water, DW).
Figure 5 represents the elution patterns of the tablet prepared in Comparative Example 3 in pH 1.2 solution, pH 4.0 solution, pH 6.8 solution and water (distilled water, DW).

### PREFERRED MODE FOR CARRYING OUT THE INVENTION

Unless explicitly described otherwise, some terms used in the present entire specification can be defined as follows.

In the present entire specification, unless mentioned especially otherwise, "comprising" or "containing" refers to comprising a certain constitutional element (or constitutional component) without special limitation, and it is not interpreted as excluding addition of other constitutional element (or constitutional component).

The present invention is explained in more detail below.

One aspect of the present invention relates to a solid dispersion comprising Olaparib; polymethacrylate copolymer; and cellulose-based polymer.

"Olaparib" may be Olaparib base (free base drug with no separate salt), or its pharmaceutically acceptable salt or its isomer, or a mixture thereof. Also, in each case it may form various hydrates, and in each case it may form various crystal forms. For example, it may be a crystal form of Olaparib hydrate (for example, monohydrate) or anhydrous Olaparib, or an amorphous form thereof, or a mixture thereof.

Olaparib may be in various forms, and for example, it may be in a micronized form in the size of several to several tens of micrometers, more preferably a micronized form in the size of several micrometers (for example, 1 to 9 micrometers size), and still more preferably a nanoparticulated form to the size of hundreds of nanometers (for example, 100 to 900 nanometers size). As the particle size of the drug becomes smaller, the dissolution rate becomes higher, and thus the solubility and bioavailability can be increased.

In an embodiment, the polymethacrylate copolymer may be a cationic polymer with dimethyl-aminoethyl methacrylate, and more concretely, it may be poly(butyl methacrylate-co-(2-dimethylaminoethyl) methacrylate-co-methyl methacrylate). The poly(butyl methacrylate-co-(2-dimethylaminoethyl) methacrylate-co-methyl methacrylate) may be a polymer comprising each of the monomers with 1:2:1 molar ratio.

As a concrete example, the polymethacrylate copolymer may be Eudragit^{®} E PO (Evonik, Germany) or Eudragit ^{®} E 100 (Evonik, Germany). Eudragit^{®} E polymer is a conventional coating base material, and used when it is needed to protect the drug from moisture, or if the patient's medication compliance is poor, for the purpose of alleviating the patient's reluctance to take by encapsulating the sensitive drug or masking the flavor and smell of the drug, and providing smooth and shiny surface so that the patient can swallow the drug easily. Whereas, in the present invention, it is used as a polymer for improving solubility and bioavailability of Olaparib.

The polymethacrylate copolymer may have a weight average molecular weight of 3,000 to 200,000, or 5,000 to 150,000, or 10,000 to 100,000, or 20,000 to 80,000, or 37,000 to 57,000 g/mole. If the weight average molecular weight of the polymethacrylate copolymer is less than 3,000 g/mole, the effect of improving water solubility may be low, and if the weight average molecular weight is greater than 200,000 g/mole, disintegration may be delayed.

There is no special limitation to the state of the polymethacrylate copolymer, but it may be in a state of granule or powder.

In an embodiment, the amount of the polymethacrylate copolymer comprised in the solid dispersion of the present invention may be, based on 1 part by weight of Olaparib, 0.05 part by weight or more, 0.1 part by weight or more, 0.15 part by weight or more, 0.2 part by weight or more, or 0.5 part by weight or more, and it also may be 10 parts by weight or less, 9 parts by weight or less, 8 parts by weight or less, 7 parts by weight or less, 6 parts by weight or less, 5 parts by weight or less, 4 parts by weight or less, 3 parts by weight or less, or 2 parts by weight or less, but it is not limited thereto.

For example, the solid dispersion of the present invention may comprise the polymethacrylate copolymer in an amount of 0.05 to 10 parts by weight, 0.1 to 6 parts by weight, 0.2 to 4 parts by weight, or 0.5 to 2 parts by weight, based on 1 part by weight of Olaparib. If the amount of the polymethacrylate copolymer in the solid dispersion is less than the above lower limit, the effect of improving solubility and bioavailability of Olaparib may be insufficient, and if it is greater than the above upper limit, the formulation (e.g., capsule or tablet) becomes too big, which may cause discomfort to patients when taking it.

In an embodiment, the cellulose-based polymer is selected from the group consisting of hydroxypropylmethylcellulose (HPMC), microcrystalline cellulose (MCC), hydroxypropylcellulose (HPC), carboxymethylcellulose (CMC), ethylcellulose (EC), cellulose acetate (CA), methylcellulose and mixtures thereof, and more concretely, it is selected from the group consisting of hydroxypropylmethylcellulose (HPMC), microcrystalline cellulose (MCC), hydroxypropylcellulose (HPC), carboxymethylcellulose (CMC) and mixtures thereof, and still more concretely, it is selected from the group consisting of hydroxypropylmethylcellulose (HPMC), hydroxypropylcellulose (HPC) and mixtures thereof, and most preferably, it is hydroxypropylmethylcellulose (HPMC).

In an embodiment, the amount of the cellulose-based polymer comprised in the solid dispersion of the present invention may be, based on 1 part by weight of Olaparib, 0.05 part by weight or more, 0.1 part by weight or more, 0.15 part by weight or more, or 0.2 part by weight or more, and it also may be 10 parts by weight or less, 9 parts by weight or less, 8 parts by weight or less, 7 parts by weight or less, 6 parts by weight or less, 5 parts by weight or less, 4 parts by weight or less, 3 parts by weight or less, 2 parts by weight or less, or 1 part by weight or less, but it is not limited thereto.

For example, the solid dispersion of the present invention may comprise the cellulose-based polymer in an amount of 0.05 to 10 parts by weight, 0.1 to 6 parts by weight, 0.2 to 4 parts by weight, or 0.2 to 1 part by weight, based on 1 part by weight of Olaparib. If the amount of the cellulose-based polymer in the solid dispersion is less than the above lower limit, the effect of improving the temperature stability of the solid dispersion may be insufficient, and if it is greater than the above upper limit, the elution becomes too delayed and the drug release in the body may be affected.

In an embodiment, the solid dispersion of the present invention may further comprise hydrophilic polymer other than the cellulose-based polymer.

The additional hydrophilic polymer as such may be, for example, one or more selected from the group consisting of polyvinylcaprolactam-polyvinyl acetate-polyethylene glycol graft copolymer (e.g., Soluplus ^{®}), polyvinylpyrrolidone (PVP), polyvinyl acetate (PVA), polyvinyl alcohol, polymer of acrylic acid or salt thereof, vinylpyrrolidone-vinylacetate copolymer (e.g., Kollidon ^{®} VA64, BASF), Polycoat IR, gelatin, guar gum, partially hydrolyzed starch, alginate, xanthan, and mixtures thereof, but it is not limited thereto.

In an embodiment, in case of using the additional hydrophilic polymer, it may be comprised in the solid dispersion, for example, in an amount of 0.01 to 10 parts by weight, 0.1 to 7 parts by weight, 0.1 to 5 parts by weight, or 0.1 to 3 parts by weight, based on 1 part by weight of Olaparib, but it is not limited thereto.

In an embodiment, the solid dispersion may further comprise D-α-tocopheryl polyethylene glycol succinate (Vitamin E TPGS or TPGS). In this case, excellent effect can be obtained for improving solubility and dissolution rate of Olaparib.

The TPGS may be comprised in the solid dispersion, for example, in an amount of 0.001 to 2 parts by weight, 0.001 to 1 part by weight, 0.005 to 1 part by weight, or 0.01 to 0.1 part by weight, based on 1 part by weight of Olaparib. If the amount of the TPGS is less than the above lower limit, the degree of improving solubility and bioavailability of Olaparib may be insufficient and thus the effect may be lowered, and if it is greater than the above upper limit, the coating layer becomes too sticky, which may cause difficulty in film formation and storage as well as preparation.

In an embodiment, the solid dispersion may be a spray-dried one. The solid dispersion is not a form of coated inactivated core, and thus it may be used in preparing tablet so that more Olaparib can be contained in a tablet having a size as small as possible, and it may prevent a phenomenon wherein the disintegration rate and elution ratio rapidly increase or decrease.

In an embodiment, the solid dispersion is amorphous. If the solid dispersion is amorphous, since the solubility increases as compared with crystal form, there is advantage of further increased *in vivo* absorption ratio.

The term "solid dispersion" used in the present invention refers to a form wherein the material to be dispersed (generally, drug) in crystal or amorphous form is dispersed in an amorphous polymer (continuous phase). More preferably, it refers to a form wherein the drug in amorphous form is dispersed in an amorphous polymer. The solid dispersion generally comprises drug and polymer, and in some cases, it may further comprise other additive(s) for drug such as surfactant, plasticizer, disintegrating agent, etc.

Thus, in a preferred embodiment, the solid dispersion comprises amorphous Olaparib.

In an embodiment, the solid dispersion does not comprise surfactant.

In an embodiment, the solid dispersion may further comprise surfactant.

The surfactant may be, for example, anionic surfactant. The anionic surfactant may be sodium dodecyl sulfate, sodium lauryl sulfate, sodium N-lauroyl sarcosylate, salt of N-long chain acyl glutamate, sucrose fatty acid ester, polyoxyethylene hydrogenated castor oil, sorbitan fatty acid ester, copolymer of polyoxyethylene and polyoxypropylene, or a combination thereof, or it may be sodium dodecyl sulfate.

The surfactant may be used, for example, in an amount of 0.0001 to 1 part by weight, or 0.0005 to 0.5 part by weight, or 0.001 to 0.1 part by weight, or 0.001 to 0.01 part by weight, or 0.001 to 0.005 part by weight, based on 1 part by weight of Olaparib.

The solvent used in a solution for preparing the solid dispersion may be water-miscible organic solvent, water or a mixture thereof. The water-miscible organic solvent may be, for example, selected from the group consisting of alcohol, acetone, tetrahydrofuran, acetic acid, acetonitrile, dioxane, and combinations thereof, but it is not limited thereto. The water-miscible organic solvent may be, for example, a lower alcohol having 1 to 5 carbon atoms, acetone, or a mixture thereof, or it may be ethanol, acetone or a mixture thereof, or it may be ethanol.

In case of solid dispersion prepared by a coating method, after cutting the cross-section of the coated particle, the thickness of the coating layer can be measured by a method such as a scanning electron microscope (SEM). At this time, the average thickness can be calculated by measuring 5 points or more and averaging them. When observing the cross-section of the coating layer for the finally coated particle, the average thickness of the coating layer may be from 10 µm to 500 µm, or from 20 µm to 400 µm, or from 50 µm to 300 µm, or from 80 µm to 200 µm. If the average thickness of the coating layer is less than the above range, the content of the drug in the particle is lowered and thus a large amount of inert core is required, which may increase the size of the formulation, and if the average thickness of the coating layer is greater than the above range, the desired purpose cannot be achieved due to delayed dissolution or excessive processing time.

In the procedure of coating as described above, various biologically inactive ingredients may be additionally used for additional purposes such as coating efficiency, drug stability, appearance, color, protection, maintenance, bonding, performance improvement, preparation process improvement, etc.

The other aspect of the present invention relates to a method for preparing a solid dispersion, the method comprising: (1) a step of dissolving or dispersing Olaparib together with polymethacrylate copolymer in a solvent; (2) a step of adding cellulose-based polymer to the solution or dispersion liquid obtained in said step (1) and mixing them; and (3) a step of removing the solvent from the solution or dispersion liquid obtained in said step (2).

In an embodiment, the solvent used to prepare the solid dispersion may be water-miscible organic solvent, water or a mixture thereof, and it may include supercritical fluid. The water-miscible organic solvent may be, for example, selected from the group consisting of alcohol, acetone, tetrahydrofuran, acetic acid, acetonitrile, dioxane, and combinations thereof, but it is not limited thereto. The water-miscible organic solvent may be, for example, a lower alcohol having 1 to 5 carbon atoms, acetone, or a mixture thereof, or it may be ethanol, acetone or a mixture thereof, or it may be ethanol.

The removal of the solvent may be conducted by using vacuum drying, spray drying, tray drying, freeze drying or other drying technique, and in an embodiment, the removal of the solvent is conducted by spray drying. In this case, for the spray drying, spray dryer, fluid bed dryer, or other dryer may be used.

The solid dispersion prepared as above may be mixed with other additional components and may be powdered or granulized, or tableted by tableting the mixture, or encapsulated by filling into a capsule.

According to the present invention, since Olaparib which is a crystalline drug is prepared as an amorphous solid dispersion, the solubility and elution rate increase and thereby bioavailability can be improved, and furthermore, the physical stability of the solid dispersion can be improved by using polymethacrylate copolymer and cellulose-based polymer together.

Another aspect of the present invention relates to an oral composition comprising the solid dispersion; pharmaceutically acceptable organic acid; and pharmaceutically acceptable diluent.

The oral composition may be used for the treatment of cancer.

In an embodiment, the cancer is selected from breast cancer and ovarian cancer.

Still another aspect of the present invention relates to a method for preparing an oral composition comprising a step of mixing the solid dispersion with pharmaceutically acceptable organic acid and pharmaceutically acceptable diluent.

The oral composition may be, for example, an oral solid formulation in tablet, capsule, granule, or powder form, and particularly it may be tablet or capsule form. In an embodiment, the oral composition is tablet.

In an embodiment, the amount of the solid dispersion in the oral composition may be, based on 1 part by weight of the oral composition, 0.01 part by weight or more, 0.05 part by weight or more, 0.1 part by weight or more, 0.15 part by weight or more, 0.2 part by weight or more, 0.25 part by weight or more, or 0.3 part by weight or more, and it also may be 0.9 part by weight or less, 0.85 part by weight or less, 0.8 part by weight or less, 0.75 part by weight or less, or 0.7 part by weight or less, but it is not limited thereto. If the oral composition comprises the solid dispersion in an amount too greater than the above range, the size of the tablet may become too large, and if the amount is too less than the above range, the formulation becomes too big, it may be difficult to stably achieve the desired *in vivo* absorption rate.

In an embodiment, the pharmaceutically acceptable organic acid is selected from the group consisting of acrylic acid, tartaric acid, citric acid, malic acid, maleic acid, fumaric acid, acetic acid, succinic acid, lactic acid, succinic acid, malonic acid, glutaric acid and mixtures thereof, and more concretely, it is selected from the group consisting of malic acid, maleic acid, tartaric acid, citric acid, fumaric acid and mixtures thereof, and still more concretely, it is selected from the group consisting of fumaric acid, maleic acid, malic acid and mixtures thereof, and most preferably, it is fumaric acid.

In an embodiment, the amount of the pharmaceutically acceptable organic acid in the oral composition may be, based on 1 part by weight of the oral composition, 0.01 part by weight or more, 0.02 part by weight or more, 0.03 part by weight or more, 0.04 part by weight or more, or 0.05 part by weight or more, and it also may be 0.5 part by weight or less, 0.45 part by weight or less, 0.4 part by weight or less, 0.35 part by weight or less, 0.3 part by weight or less, or 0.2 part by weight or less, but it is not limited thereto. If the oral composition comprises the pharmaceutically acceptable organic acid in an amount too greater than the above range, the stability of the formulation may be affected, and if the amount is too less than the above range, the stability of the formulation according to pH change may deteriorate.

The pharmaceutically acceptable diluent may be water-soluble diluent or water-insoluble diluent, and it may be, for example, one or more selected from the group consisting of lactose (anhydrous or hydrated, e.g. monohydrate), cellulose powder, microcrystalline cellulose, silicified microcrystalline cellulose, dicalcium phosphate, tricalcium phosphate, magnesium trisilicate, low-substituted hydroxypropylcellulose (L-HPC), starch, pregelatinized starch, mannitol, maltitol, sorbitol, xylitol, lactose, dextrose, maltose, sucrose, glucose, fructose, maltodextrin and mixtures thereof, but it is not limited thereto.

In an embodiment, the pharmaceutically acceptable diluent is water-soluble diluent.

In an embodiment, the water-soluble diluent is selected from the group consisting of sugar alcohols, saccharides and mixtures thereof.

In an embodiment, the sugar alcohol may be mannitol, maltitol, sorbitol, xylitol or a combination thereof, and the saccharide may be lactose, dextrose, maltose, sucrose, glucose, fructose, maltodextrin or a combination thereof.

In an embodiment, the water-insoluble diluent may be one or more selected from the group consisting of microcrystalline cellulose, silicified microcrystalline cellulose, dicalcium phosphate, tricalcium phosphate, low-substituted hydroxypropylcellulose, starch, pregelatinized starch and mixtures thereof.

In an embodiment, the oral composition may not comprise the water-insoluble diluent.

In an embodiment, the amount of the pharmaceutically acceptable diluent in the oral composition may be, based on 1 part by weight of the oral composition, 0.01 part by weight or more, 0.03 part by weight or more, 0.05 part by weight or more, 0.07 part by weight or more, or 0.1 part by weight or more, and it also may be 0.5 part by weight or less, 0.45 part by weight or less, 0.4 part by weight or less, 0.35 part by weight or less, or 0.3 part by weight or less, but it is not limited thereto. If the oral composition comprises the pharmaceutically acceptable diluent in an amount too greater than the above range, the tablet becomes too big, which may cause discomfort to patients when taking it, and if the amount is too less than the above range, it is difficult to formulate into tablets due to low tableting properties.

The oral composition of the present invention may further comprise one or more pharmaceutically acceptable additives other than the above-explained components (i.e., the pharmaceutically acceptable organic acid and the pharmaceutically acceptable diluent).

The one or more pharmaceutically acceptable further additives may be, for example, disintegrating agent, lubricant, surfactant, coating agent, or a combination thereof.

The disintegrating agent may be, for example, one or more selected from the group consisting of croscarmellose sodium (CrosCMC-Na), carboxymethylcellulose, crospovidone (crosslinked polyvinylpyrrolidone), L-HPC (low-substituted hydroxypropylcellulose), starch (wheat, rice, corn or potato starch), sodium carboxymethyl starch, sodium glycolate of potato starch, partially hydrolyzed starch, and mixtures thereof, but it is not limited thereto. Preferably, it may be croscarmellose sodium (CrosCMC-Na) or sodium glycolate of starch, L-HPC (low-substituted hydroxypropyl cellulose) or a mixture thereof.

In an embodiment, the disintegrating agent may be used in an amount of, for example, 1 to 30 parts by weight, and preferably 2 to 20 parts by weight, based on 100 parts by weight of the total tablet weight. If the amount of the disintegrating agent is less than the above lower limit, there may be a problem of elution delay due to disintegration delay, and if the amount is greater than the above upper limit, there may be a problem with productivity such as tableting disorder.

The lubricant may be, for example, one or more selected from the group consisting of magnesium stearate, fumaric acid, stearic acid, calcium stearate, sodium stearyl fumarate, polyethylene glycol, starch (wheat, rice, corn or potato starch), talc, highly dispersed (colloidal) silica, magnesium oxide, magnesium carbonate, glyceryl behenate, glyceryl monostearate, silicon dioxide, calcium silicate, magnesium silicate and mixtures thereof, but it is not limited thereto. Preferably, it may be magnesium stearate.

In an embodiment, the lubricant may be used in an amount of, for example, 0.1 to 3 parts by weight, preferably 0.2 to 3 parts by weight, and more preferably 0.5 to 2 parts by weight, based on 100 parts by weight of the total tablet weight. If the amount of the lubricant is less than the above lower limit, there may be a problem with productivity such as tableting disorder, and if the amount is greater than the above upper limit, there may be elution delay or a problem with productivity.

The surfactant may be, for example, one or more selected from the group consisting of anionic surfactant (sodium lauryl sulfate, sodium docusate, etc.), cationic surfactant (cetyltrimethylammonium bromide (CTAB), benzethonium chloride, etc.), nonionic surfactant (polysorbates 80, 40 and 20, lauryldimethylamine N-oxide (LDAO), etc., Cremophor RH40^{™}, polyoxyethylene stearate and poloxamer and mixtures thereof, but it is not limited thereto. Preferably, it may be sodium lauryl sulfate.

In an embodiment, the surfactant may be used in an amount of, for example, 0.1 to 10 parts by weight, preferably 0.5 to 7 parts by weight, and more preferably 1 to 5 parts by weight, based on 100 parts by weight of the total formulation weight. If the amount of the surfactant is less than the above lower limit, it is difficult to see sufficient effect of increasing solubility, and if the amount is greater than the above upper limit, the formulation stability may be affected.

The coating agent may be, for example, as hydrophilic polymer, one or more selected from the group consisting of polyvinylpyrrolidone (PVP), polyvinyl acetate (PVA), hydroxypropylmethylcellulose (HPMC), carboxymethylcellulose (sodium salt and calcium salt), ethylcellulose, methylcellulose, hydroxyethylcellulose, ethylhydroxyethylcellulose, hydroxypropylcellulose (HPC), L-HPC (low substituted HPC), polyvinyl alcohol, polymer of acrylic acid or salt thereof, vinyl pyrrolidone-vinyl acetate copolymer (e.g., Kollidon) (registered trademark) VA64, BASF), Polycoat IR, gelatin, guar gum, partially hydrolyzed starch, alginate, xanthan, and mixtures thereof, but it is not limited thereto. Preferably, it may be polyvinyl acetate (PVA) or hydroxypropylmethylcellulose (HPMC).

In an embodiment, the coating agent may be used in an amount of, for example, 0.2 to 10 parts by weight, preferably 1 to 7 parts by weight, more preferably 3 to 5 parts by weight, based on 100 parts by weight of the tablet before coating (uncoated tablet). If the amount of the coating agent is less than the above lower limit, there may be a problem of incomplete coverage of entire surface of uncoated tablet with the coating agent, and if the amount is greater than the above upper limit, there may be a problem of excessive delay of elution.

Preferable examples are provided below in order to facilitate understanding of the present invention. However, the following examples are only to illustrate the present invention, and the scope of the present invention is not limited thereby in any manner.

### [EXAMPLES]

### Example 1: Preparation of solid dispersion of Olaparib/Eudragit E/HPMC (weight ratio of 1:1.5:0.25)

2.0g of Olaparib hydrate (crystal form), 3.0g of Eudragit E PO, and 0.1g of TPGS were dissolved completely in 200ml of EtOH. After dissolving 0.5g of HPMC completely in 20ml of water, the resulting solution was mixed uniformly with the solution in which Olaparib and Eudragit E were dissolved. The resulting mixture solution was spray dried to remove the solvent and obtain a solid dispersion.

### Example 2: Preparation of solid dispersion of Olaparib/Eudragit E/HPMC (weight ratio of 1:1:1)

2.0g of Olaparib hydrate (crystal form), 2.0g of Eudragit E PO, and 0.1g of TPGS were dissolved completely in 200ml of EtOH. After dissolving 2.0g of HPMC completely in 80ml of water, the resulting solution was mixed uniformly with the solution in which Olaparib and Eudragit E were dissolved. The resulting mixture solution was spray dried to remove the solvent and obtain a solid dispersion.

### Comparative Example 1: Preparation of solid dispersion of Olaparib and Eudragit E (weight ratio of 1:2)

2.0g of Olaparib hydrate (crystal form), 4.0g of Eudragit E PO, and 0.1g of TPGS were dissolved completely in 200ml of EtOH. The resulting solution was spray dried to remove the solvent and obtain a solid dispersion.

### Test Example 1: Solubility measurement

### <Comparison of drug solubility of Olaparib raw material and solid dispersion>

To 150 ml of pH 1.2 buffer solution, each of the solid dispersions of Examples 1 and 2 and Comparative Example 1 in an amount corresponding to 50 mg of Olaparib was added, and shaken in an incubator at 37°C with 100 rpm for 1 hour. The test solution was filtered with RC syringe filter (0.2 µm) and 1 mL of the filtered solution was taken and diluted with 2 mL of a diluent solution, and then the amount was measured according to Olaparib amount analysis method. The results are shown in Table 1 below.

**[Table 1] Results of solubility measurement**

| Sample | Solubility (mg/ml) |
|---|---|
| | pH 1.2 |
| Olaparib raw material | 0.10 |
| Example 1 | 0.33 |
| Example 2 | 0.31 |
| Comparative Example 1 | 0.32 |

### Test Example 2: Crystallinity evaluation by XRPD analysis

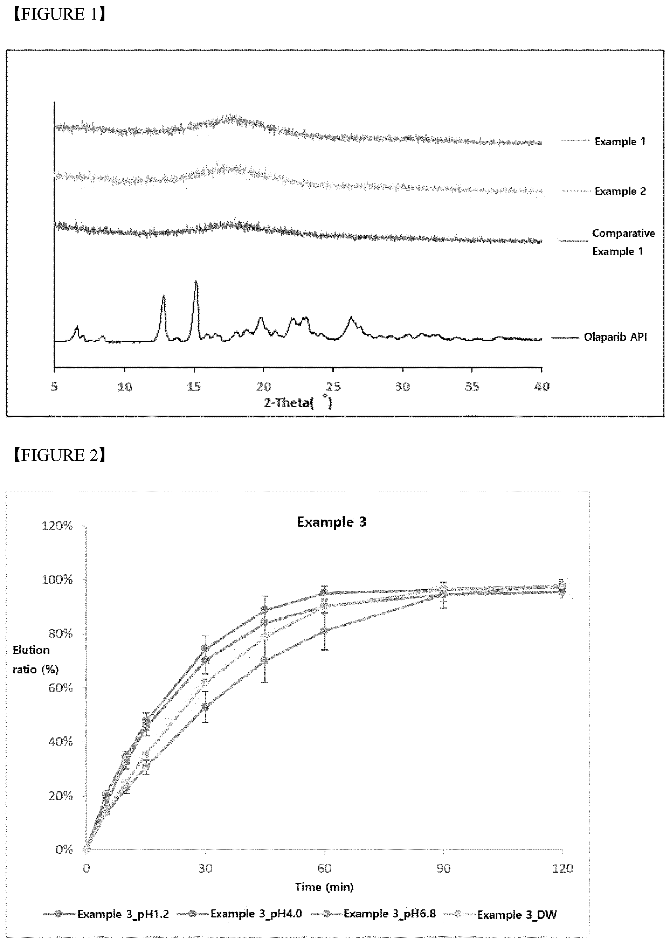

Each of the solid dispersions of Examples 1 and 2 and Comparative Example 1 was analyzed by XRPD to evaluate the crystallinity of Olaparib in the solid dispersion. XRPD patterns of each of the solid dispersions prepared in Examples 1 and 2 and Comparative Example 1 and Olaparib API (raw material active ingredient) were shown in Figure 1. The XRPD patterns in Figure 1 show that Olaparib API itself used in preparation of the solid dispersions was crystalline, whereas all Olaparib contained in the solid dispersions of Examples 1 and 2 and Comparative Example 1 were amorphous.

### Test Example 3: Stability

Each of the solid dispersions of Examples 1 and 2 and Comparative Example 1 was filled in a hard gelatin capsule in an amount corresponding to 150 mg of Olaparib, and the capsule was put into an aluminum bag pouch together with a drying agent and sealed, and then stored for 6 months under storage condition of 40°C and 75% RH as accelerated condition. Elution ratio was measured before storing the sample and at the time of 6 months after the storage in order to observe the elution ratio change.

### Elution condition

For the hard gelatin capsule filled with Olaparib (150 mg as Olaparib), an elution test was conducted under the condition below. A sample was taken at the time of 60 minutes and the elution ratio of the drug was measured through HPLC analysis.
Test solution: pH 1.2 buffer 450 ml
Stirring speed: 100 rpm
Temperature: 37°C
Points of time for taking the sample: 5, 10, 15, 30, 45, 60 minutes

**[Table 2] Results of stability test**

| Sample | | Elution ratio of Olaparib according to time | | | | | |
|---|---|---|---|---|---|---|---|
| | | 5 minutes | 10 minutes | 15 minutes | 30 minutes | 45 minutes | 60 minutes |
| Example 1 | Initial | 17.9% | 38.1% | 55.4% | 82.7% | 92.2% | 93.6% |
| | After 6 months storage | 22.8% | 48.7% | 65.7% | 92.5% | 97.2% | 97.2% |
| Example 2 | Initial | 2.3% | 13.0% | 31.7% | 79.3% | 86.7% | 93.1% |
| | After 6 months storage | 1.9% | 10.2% | 23.2% | 63.1% | 88.3% | 93.5% |
| Comparative Example 1 | Initial | 21.1% | 36.7% | 48.8% | 80.8% | 94.7% | 100.8% |
| | After 6 months storage | 15.0% | 30.3% | 44.6% | 66.4% | 80.4% | 80.7% |

From the results in Table 2 above, it was confirmed that the solid dispersion of Comparative Example 1 prepared with Olaparib and Eudragit E only exhibited a remarkably reduced stability in the accelerated test.

### Example 3: Preparation of Olaparib tablet (prototype)

To the solid dispersion of Example 1, mannitol, fumaric acid, croscarmellose sodium and magnesium stearate were added according to the following composition to prepare Olaparib tablet.

| Raw material | | Tablet of Example 3 (mg) |
|---|---|---|
| Solid dispersion (Example 1) | Olaparib hydrate | 156.2(150.0 as Olaparib) |
| | TPGS | 7.8 |
| | Eudragit E | 234.3 |
| | HPMC | 39.1 |
| Mannitol | | 141.0 |
| Fumaric acid | | 70 |
| Croscarmellose sodium | | 27.0 |
| Magnesium stearate | | 6.0 |
| Total | | 681.4 |

### Example 4: Preparation of Olaparib tablet (prototype)

To the solid dispersion of Example 1, mannitol, fumaric acid, croscarmellose sodium and magnesium stearate were added according to the following composition to prepare Olaparib tablet.

| Raw material | | Tablet of Example 3 (mg) |
|---|---|---|
| Solid dispersion (Example 1) | Olaparib hydrate | 156.2(150.0 as Olaparib) |
| | TPGS | 7.8 |
| | Eudragit E | 234.3 |
| | HPMC | 39.1 |
| Mannitol | | 91.0 |
| Fumaric acid | | 70 |
| Croscarmellose sodium | | 27.0 |
| Magnesium stearate | | 6.0 |
| Total | | 631.4 |

### Comparative Example 2: Preparation of Olaparib tablet (prototype)

To the solid dispersion of Example 1, microcrystalline cellulose, fumaric acid, croscarmellose sodium and magnesium stearate were added according to the following composition to prepare Olaparib tablet.

| Raw material | | Tablet of Example 3 (mg) |
|---|---|---|
| Solid dispersion (Example 1) | Olaparib hydrate | 156.2(150.0 as Olaparib) |
| | TPGS | 7.8 |
| | Eudragit E | 234.3 |
| | HPMC | 39.1 |
| Microcrystalline cellulose | | 170.0 |
| Fumaric acid | | 50 |
| Croscarmellose sodium | | 18.0 |
| Magnesium stearate | | 6.0 |
| Total | | 681.4 |

### Comparative Example 3: Preparation of Olaparib tablet (prototype)

To the solid dispersion of Example 1, mannitol, croscarmellose sodium and magnesium stearate were added according to the following composition to prepare Olaparib tablet.

| Raw material | | Tablet of Example 3 (mg) |
|---|---|---|
| Solid dispersion (Example 1) | Olaparib hydrate | 156.2(150.0 as Olaparib) |
| | TPGS | 7.8 |
| | Eudragit E | 234.3 |
| | HPMC | 39.1 |
| Mannitol | | 141.0 |
| Croscarmellose sodium | | 27.0 |
| Magnesium stearate | | 6.0 |
| Total | | 611.4 |

### Test Example 4: Elution test of Olaparib tablet

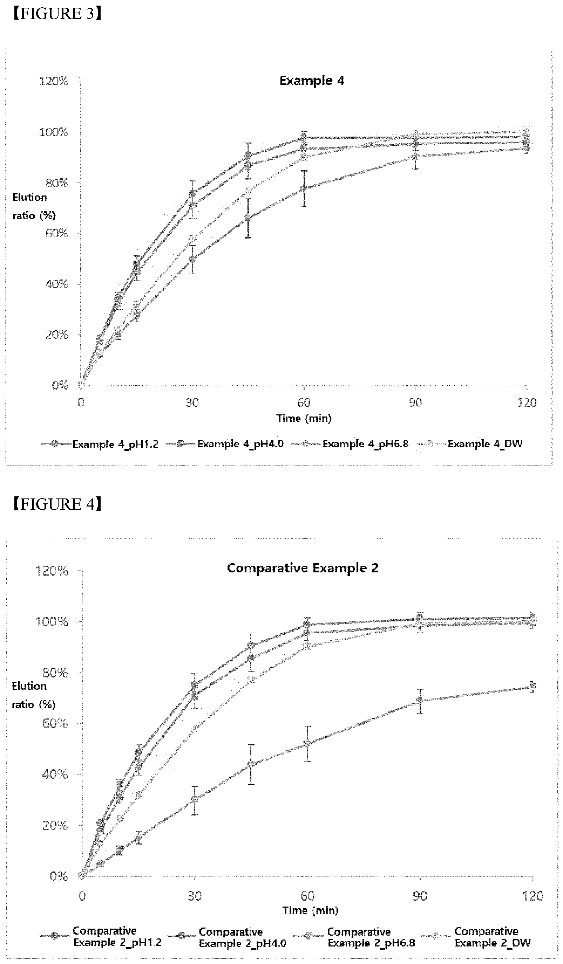

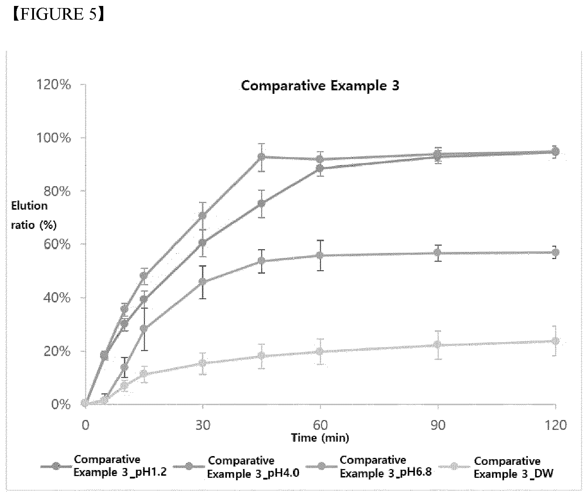

To the tablets of Examples 3 and 4 and Comparative Examples 2 and 3, elution ratios according to time in buffer solutions of pH 1.2, pH 4.0 and pH 6.8 and water (distilled water, DW) were measured, and the results are shown in Figures 2, 3, 4 and 5, respectively. From Figures 2 to 5, it can be confirmed that, in comparison with the tablets of Comparative Examples, the tablets of Examples comprising the solid dispersion according to the present invention were eluted more uniformly in all test solution conditions.

### Elution condition

Test solution: Buffer solutions of pH 1.2, pH 4.0 and pH 6.8 and water (distilled water, DW) 450ml
Stirring speed: 100 rpm
Temperature: 37°C
Standard time of elution: 5 minutes, 10 minutes, 15 minutes, 30 minutes, 45 minutes, 60 minutes, 90 minutes, 120 minutes
Analysis method: HPLC method

### Test Example 5: Pharmacokinetic (PK) test of Olaparib tablet

In order to compare *in vivo* behavior of the tablets of Examples 3 and 4 and Comparative Example 2 (formulations containing Olaparib solid dispersion), pharmacokinetic test was conducted with beagle dogs as test animal. Beagle dogs were divided into three (3) groups and were administered with the formulation. Each formulation comprised 150mg of Olaparib, and a dose of 1 tablet/head together with 20ml of water was administered orally to the test animals maintained in a fasted state. After the administration, the blood samples were taken with predetermined time interval up to 24 hours, and serums were separated from the blood samples and frozen for storage, and analyzed with LC/MS/MS device for concentration analysis to measure the drug concentrations in blood according to time. From the data, AUC (area under serum concentration-time curve) and Cₘₐₓ (the maximum concentration in blood) were obtained, and the results are shown in Table 3 below.

**[Table 3] Results of pharmacokinetic (PK) test of Olaparib solid dispersion tablet**

| | AUC (ng/ml•h) | Cₘₐₓ (ng/ml) |
|---|---|---|
| Example 3 | 43524.2±12977.8 | 8650.0±2201.8 |
| Example 4 | 39259.6±17209.1 | 8440.0±3776.8 |
| Comparative Example 2 | 33589.9±4863.9 | 7020.0±1360.7 |

From the results of pharmacokinetic test in Table 3 above, it can be known that the formulations of Examples 3 and 4 comprising the Olaparib solid dispersion according to the present invention improved the solubility and *in vivo* absorption of the raw material Olaparib and thus exhibited AUC and Cₘₐₓ in preferable ranges, whereas those of the formulation of Comparative Example 2 could not fall within the pharmacokinetically preferable ranges.

## Claims

1. A solid dispersion comprising Olaparib; polymethacrylate copolymer; and cellulose-based polymer.

2. The solid dispersion of claim 1, wherein the polymethacrylate copolymer is a cationic polymer with dimethyl-aminoethyl methacrylate.

3. The solid dispersion of claim 1, wherein the polymethacrylate copolymer is poly(butyl methacrylate-co-(2-dimethylaminoethyl) methacrylate-co-methyl methacrylate).

4. The solid dispersion of claim 1, wherein the cellulose-based polymer is selected from the group consisting of hydroxypropylmethylcellulose, microcrystalline cellulose, hydroxypropylcellulose, carboxymethylcellulose, ethylcellulose, cellulose acetate, methylcellulose and mixtures thereof.

5. The solid dispersion of claim 1, which further comprises D-α-tocopheryl polyethylene glycol succinate.

6. The solid dispersion of claim 1 which is obtained by spray drying.

7. The solid dispersion of claim 1, which comprises amorphous Olaparib.

8. A method for preparing a solid dispersion, the method comprising:
(1) a step of dissolving or dispersing Olaparib together with polymethacrylate copolymer in a solvent;
(2) a step of adding cellulose-based polymer to the solution or dispersion liquid obtained in said step (1) and mixing them; and
(3) a step of removing the solvent from the solution or dispersion liquid obtained in said step (2).

9. The method for preparing a solid dispersion of claim 8, wherein the removal of the solvent is conducted by spray drying.

10. An oral composition comprising a solid dispersion of any one of claims 1 to 7; pharmaceutically acceptable organic acid; and pharmaceutically acceptable diluent.

11. The oral composition of claim 10, wherein the pharmaceutically acceptable organic acid is selected from the group consisting of acrylic acid, tartaric acid, citric acid, malic acid, maleic acid, fumaric acid, acetic acid, succinic acid, lactic acid, succinic acid, malonic acid, glutaric acid and mixtures thereof.

12. The oral composition of claim 10, wherein the pharmaceutically acceptable diluent is water-soluble diluent.

13. The oral composition of claim 12, wherein the water-soluble diluent is selected from the group consisting of sugar alcohols, saccharides and mixtures thereof.

14. The oral composition of claim 10, which is for treatment of cancer.

15. A method for preparing an oral composition comprising a step of mixing a solid dispersion of any one of claims 1 to 7 with pharmaceutically acceptable organic acid and pharmaceutically acceptable diluent.
